(19) Europäisches Patentamt
European Patent Office
Office européen des brevets

(11) Veröffentlichungsnummer: **0 049 499**
**B1**

(12) # EUROPÄISCHE PATENTSCHRIFT

(45) Veröffentlichungstag der Patentschrift: **29.01.86**

(51) Int. Cl.⁴: **C 07 D 501/36**, A 61 K 31/545

(21) Anmeldenummer: **81107866.6**

(22) Anmeldetag: **02.10.81**

(54) **Neue Cephalosporinderivate, Verfahren zu ihrer Herstellung und Zwischenprodukte dafür sowie entsprechende pharmazeutische Präparate.**

(30) Priorität: **06.10.80 CH 7450/80**

(43) Veröffentlichungstag der Anmeldung: **14.04.82 Patentblatt 82/15**

(45) Bekanntmachung des Hinweises auf die Patenterteilung: **29.01.86 Patentblatt 86/5**

(84) Benannte Vertragsstaaten: **AT BE CH DE FR GB IT LI LU NL SE**

(56) Entgegenhaltungen:
**FR - A - 2 385 722**
**FR - A - 2 408 612**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Reiner, Roland, Dr., Rheinfelderstrasse 8, CH-4058 Basel (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 049 499 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Acylderivate, und zwar Cephalosporinderivate der allgemeinen Formel

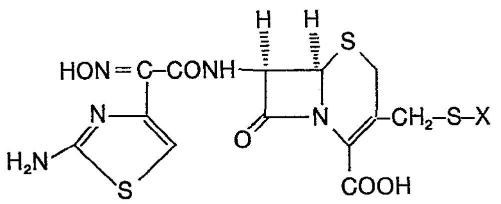

I

in der X die
1,2,5,6-Tetrahydro-2-methyl-5,6-dioxo-as-triazin-3-ylgruppe
bzw. deren entsprechende tautomere Form, die
2,5-Dihydro-6-hydroxy-2-methyl-5-oxo-as-triazin-3-ylgruppe,
darstellt,
sowie leicht hydrolysierbare Ester, leicht hydrolysierbare Äther und Salze dieser Verbindungen und Hydrate der Verbindungen der Formel I bzw. von deren Estern, Äthern und Salzen.

Als leicht hydrolysierbare Ester der Verbindungen der Formel I sind Verbindungen der Formel I zu verstehen, deren Carboxygruppen in Form einer leicht hydrolysierbaren Estergruppe vorliegt. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl und 1-Pivaloyloxyäthylester; die niederen Alkoxycarbonyloxyalkylester, z.B. der Methoxycarbonyloxymethyl-, 1-Äthoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester. z.B. der Acetamidomethylester. Auch andere Ester, z.B. die Benzyl- und Cyanmethylester, können brauchbar sein.

Als leicht hydrolysierbare Äther der Verbindungen der Formel I sind Verbindungen der Formel I zu verstehen, worin X die 2,5-Dihydro-6-hydroxy-2-methyl-5-oxo-as-triazin-3-ylgruppe bedeutet, deren enolische OH-Gruppe in Form einer leicht hydrolysierbaren Äthergruppe vorliegt. Als Äthergruppen kommen die gleichen Gruppen in Betracht, wie sie oben bereits für die leicht hydrolysierbaren Estergruppen erwähnt wurden. Vertreter solcher Äther sind also beispielsweise die niederen Alkanoyloxyalkyläther, z.B. der Acetoxymethyl-, Pivaloyloxymethyl-, 1-Acetoxyäthyl- und 1-Pivaloyloxyäthyläther; die niederen Alkoxycarbonyloxyalkyläther, z.B. der Methoxycarbonyloxymethyl-, 1-Aethoxycarbonyloxyäthyl und 1-Isopropoxycarbonyloxyäthyläther; die Lactonyläther, z.B. der Phthalidyl- und Thiophthalidyläther; die niederen Alkoxymethyläther, z.B. der Methoxymethyläther; und die niederen Alkanoylaminomethyläther, z.B. der Acetamidomethyläther.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Äthyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Monosalze oder auch Disalze sein. Die zweite Salzbildung kann in Verbindungen mit dem Hydroxyrest der
2,5-Dihydro-6-hydroxy-2-methyl-5-oxo-as-triazin-3-ylgruppe
auftreten.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate u.dgl., Alkyl- und Mono-arylsulfonate, wie Äthansulfonate, Toluolsulfonate, Benzolsulfonate u.dgl. und auch andere organische Säuresalze, wie Acetate, Tartrate, Maleate, Citrate, Benzoate, Salicylate, Ascorbate u.dgl.

Die Verbindungen der Formel I (einschliesslich deren Salze, leicht hydrolysierbare Ester und Äther) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der synisomeren Form

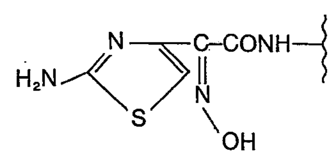

oder in der anti-isomeren Form

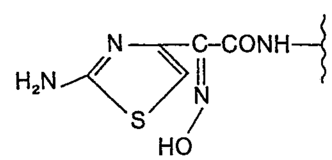

bzw. als Gemische dieser beiden Formen vorliegen. Bevorzugt ist die syn-isomere Form bzw. Gemische, in denen die syn-isomere Form überwiegt.

Bevorzugte Produkte sind
(6R, 7R)-7-[2-(2-Amino-4-thiazolyl)-2-(Z-hydroxyimino)-acetamidol]-3-/[(2,5-dihydro-6-hydroxy-2-methyl-5-oxo-as-triazin-3-yl)-thio]-methyl/-8-oxo-5-thia-1-azabicyclo-[4.2.0]oct-2-en-2-carbonsäure
und deren Salze sowie die entsprechenden Hydrate.

Die obigen Acylderivate werden erfindungsgemäss dadurch hergestellt, dass man

a) eine Verbindung der allgemeinen Formel

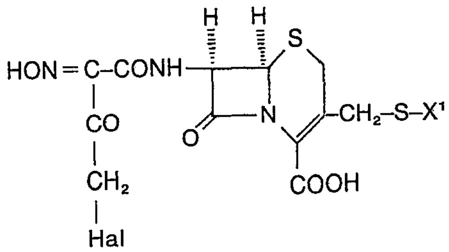

II

in der $X^1$ dieselbe Bedeutung wie X hat, wobei jedoch die 2,5-Dihydro-6-hydroxy-2-methyl-5-oxo-as-triazin-3-ylgruppe zu einem leicht hydrolysierbaren Äther veräthert sein kann, Hal Brom oder Chlor darstellt und die Carboxygruppe in geschützter Form vorliegen kann,
mit Thioharnstoff umsetzt und eine allenfalls vorhandene Carboxyschutzgruppe abspaltet, oder dass man

b) zur Herstellung eines leicht hydrolysierbaren Esters bwz. Äthers einer Verbindung der Formel I eine Carbonsäure bzw. ein Enol der Formel I einer entsprechenden Veresterung bzw. Verätherung unterwirft, oder dass man

c) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

Die in der Ausgangsverbindung der Formel II vorhandene Carboxygruppe kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden.

Die Ausgangsverbindungen der Formel II können z.B. durch N-Acylierung der entsprechenden 7-Aminoverbindung hergestellt werden, und zwar indem man eine Verbindung der allgemeinen Formel

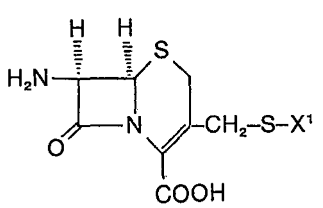

III

in der $X^1$ die oben gegebene Bedeutung hat und die Carboxygruppe und/oder die Aminogruppe in geschützter Form vorliegen kann,
mit Diketen und dem entsprechenden Halogen (Brom oder Chlor) in das entsprechende 4-Halogenacetoacetamidoderivat der allgemeinen Formel

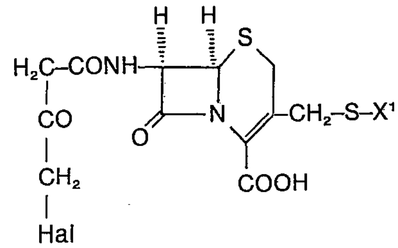

IV

worin $X^1$ und Hal die oben gegebene Bedeutung haben, überführt und dieses Produkt mit einem Nitrosierungsmittel behandelt.

Die in der 7-Aminoverbindung der Formel III vorhandene Carboxygruppe kann wahlweise geschützt sein, und zwar in der oben für die herzustellende Ausgangsverbindung der Formel II erläuterten Weise. Die Aminogruppe der Verbindung der Formel III kann z.B. durch eine Silylschutzgruppe, wie Trimethylsilyl, geschützt sein.

Die für die Herstellung der Verbindungen IV aus einer Verbindung III benötigten Agenzien Diketen bzw. Brom (oder Chlor) werden vorzugsweise in äquimolaren Mengen angewendet. Die Reaktion erfolgt tunlichst in einem inerten organischen Lösungsmittel, z.B. in Methylenchlorid, Chloroform, Tetrahydrofuran oder Mischungen davon bei niederer Temperatur, z.B. bei −50 °C bis 0 °C. Die anschliessende Nitrosierung der erhaltenen Verbindung der Formel IV kann durch Behandeln mit salpetriger Säure bzw. deren Ester, z.B. Methyl-, Äthyl- oder Amylnitrit, oder Nitrosylchlorid erfolgen. Die Reaktion erfolgt vorzugsweise in einem inerten Lösungsmittel, z.B. Wasser, Essigsäure, Dioxan, Tetrahydrofuran, Acetonitril oder Gemische hiervon, bei einer Temperatur zwischen etwa −20 °C und 50 °C, vorzugsweise bei Raumtemperatur. Unter diesen Gegebenheiten erhält man die Ausgangsverbindung der Formel II in der syn-Form (Z-Form) bzw. in Gemischen, worin die syn-Form überwiegt.

Die erfindungsgemässe Umsetzung des Halogenids der Formel II bzw. eines Salzes davon mit Thioharnstoff (Verfahrensvariante a)) erfolgt vorzugsweise in einem inerten Lösungsmittel, wie z.B. in einem niederen Alkanol, z.B. Äthanol, in einem niederen Keton, wie Aceton, in einem Äther, wie Tetrahydrofuran, Dioxan, in Dimethylformamid, Dimethylacetamid, in Wasser oder in Mischungen davon. Die Reaktionstemperatur liegt im allgemeinen im Bereich von etwa 0 °C bis 60 °C, vorzugsweise bei Zimmertemperatur. Es kann die freie Säure der Formel II eingesetzt werden, wahlweise aber auch ein Salz davon, wobei die gleichen Salze wie die oben erläuterten Salze der Verbindungen der Formel I in Betracht kommen.

Nach Durchführung der Verfahrensvariante a), kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lactonyl-, Alkoxymethyl- und Alkanoylaminomethylester werden vorzugsweise enzymatisch mit Hilfe einer geeigneten Esterase (bei etwa 20–40 °C) abgespalten. Wenn die Carboxylgruppe durch Salzbildung (z. B. mit Triäthylamin) geschützt ist, so kann die Abspaltung dieser salzbildenden Schutzgruppe durch Behandlung mit Säure erfolgen. Als Säure kann hierbei z. B. Salzsäure, Schwefelsäure, Phosphorsäure oder Citronensäure verwendet werden.

Zur Herstellung der leicht hydrolysierbaren Ester der Carbonsäuren der Formel I gemäss Variante b), wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z. B. einem Alkalimetallhydroxid oder -carbonat oder einem organischen Amin, wie Triäthylamin, beschleunigt werden. Bei Vorhandensein der
2,5-Dihydro-6-hydroxy-2-methyl-5-oxo-as-triazin-3-ylgruppe X
mit ihrer enolischen Funktion wird diese unter Bildung eines entsprechenden, leicht hydrolysierbaren Äthers veräthert. Vorzugsweise verwendet man dabei einen Überschuss an dem entsprechenden Halogenid. Die Veresterungs-/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0–40 °C.

Dei Herstellung der Salze und Hydrate der Verbindungen der Formel I bzw. der Hydrate dieser Salze kann in an sich bekannter Weise erfolgen, z. B. durch Umsetzung der Carbonsäure der Formel I mit einer äquivalenten Menge der gewünschten Base, zweckmässig in einem Lösungsmittel, wie Wasser oder in einem organischen Lösungsmittel, wie Äthanol, Methanol, Aceton und anderen mehr. Bei Verwendung eines zweiten Äquivalents an Base erfolgt Salzbildung auch an einer allenfalls vorhandenen tautomeren Enolform
(2,5-Dihydro-6-hydroxy-2-methyl-5-oxo-as-triazin-3-ylgruppe X),
wobei ein Disalz entsteht. Die Temperatur der Salzbildung ist nicht kritisch. Sie liegt im allgemeinen bei Raumtemperatur, kann aber auch leicht darüber oder darunter, etwa im Bereiche von 0 °C bis +50 °C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Äther oder Salz davon) einer feuchten Atmosphäre, z. B. bei etwa +10 °C bis +40 °C, ausgesetzt werden.

Die oben verwendeten 7-Aminoverbindungen der Formel III können ausgehend von einer Verbindung der Formel

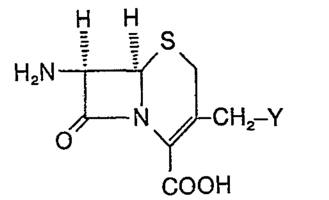
V

in der Y eine austretende Gruppe darstellt und die Carboxygruppe durch Salzbildung mit einer anorganischen oder tertiären organischen Base geschützt sein kann,
in Gegenwart von Wasser mit einem Thiol der allgemeinen Formel

$$HS\text{–}X^1 \qquad VI$$

in der $X^1$ die oben gegebene Bedeutung hat, hergestellt werden. Diese Reaktion kann in an sich bekannter Weise, z. B. bei einer Temperatur zwischen etwa 40 und 80 °C, zweckmässig bei etwa 60 °C, in Wasser oder in einer Pufferlösung mit einem pH-Wert von etwa 6 bis 7, vorzugsweise 6,5, durchgeführt werden.

Die Carboxygruppe und/oder die Aminogruppe der erhaltenen Verbindung III können erwünschtenfalls geschützt werden, z. B. durch Veresterung oder Salzbildung der Carboxygruppe oder durch Silylierung.

Die Thiole der Formel VI stehen in tautomerem Gleichgewicht mit den entsprechenden Thionen im Sinne der nachstehenden Formeln

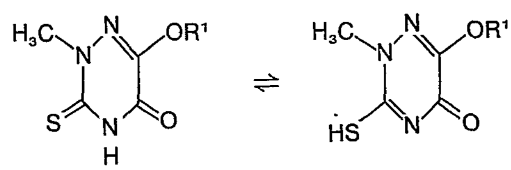
VI $a^1$ VI $a^2$

worin $R^1$ Wasserstoff oder (zusammen mit dem Sauerstoff) eine leicht hydrolysierbare Äthergruppe darstellt.

Wenn in den Formeln VI $a^1$ und VI $a^2$ $R^1$ Wasserstoff darstellt, stehen diese 6-Hydroxyverbindungen in tautomerem Gleichgewicht mit den entsprechenden 6-Oxoverbindungen im Sinne der nachstehenden Formeln:

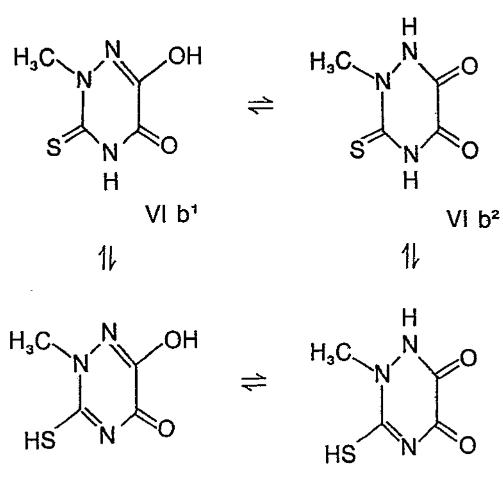
VI $b^1$ VI $b^2$

VI $b^3$ VI $b^4$

Die Herstellung von in 6-Stellung verätherten Thiolen (Thionen) der Formel VI wird in Beispiel 2 beschrieben. Im allgemeinen wird der Ätherrest eingeführt, indem man ein S-geschütztes Thiol (z.B. durch Benzhydryl) mit dem die Äthergruppe enthaltenden Halogenid, vorzugsweise dem Jodid, in einem inerten organischen Lösungsmittel in Gegenwart eines säurebindenden Mittels, z.B. Kaliumcarbonat, umsetzt, vorzugsweise bei etwa 10 °C – 50 °C, und die Schutzgruppe abspaltet (Benzhydryl kann mit Anisol und Trifluoressigsäure bei Raumtemperatur abgespalten werden).

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I sowie die entsprechenden leicht hydrolysierbaren Ester, Äther und Salze bzw. die Hydrate dieser Produkte sind antibiotisch, insbesondere bakterizid wirksam. Sie besitzen ein breites Wirkungsspektrum gegen Gram-positive und Gram-negative Mikroorganismen, einschliesslich β-Lactamase-bildende Staphylokokken und verschiedene β-Lactamase-bildende Gram-negative Bakterien, wie z.B. Pseudomonas aeruginosa, Haemophilus influenzae, Escherichia coli, Serratia marcescens, Proteus-, Neisseria- und Klebsiella-Spezies. Ein besonderes Merkmal ist die überraschend hohe Halbwertzeit der Produkte in vivo (Zeit für die Herabsetzung des Wirkstofftiters im Blutplasma auf die Hälfte), welche den gewichtigen Vorteil mit sich bringt, dass, abgesehen von Unterschieden in der spezifischen bakteriziden Wirkung im Vergleich zu anderen Produkten mit niedrigeren Halbwertzeiten, eine geringere Wirkstoffmenge für die Kontrolle einer entsprechenden Infektion benötigt wird; ferner können für die Aufrechterhaltung eines gewünschten Minimaltiters an Wirkstoff im Blut längere Intervalle zwischen den Verabreichungen erlaubt werden.

Die Verbindungen der Formel I sowie die entsprechenden leicht hydrolysierbaren Ester, Äther und Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erfindungsgemässen Produkte wurden repräsentative Vertreter gegen verschiedene Krankheitserreger getestet. Folgende Produkte wurden geprüft:

Produkt A:
(6R, 7R)-7-[2-(2-Amino-4-thiazolyl)-2-(Z-hydroxyimino)acetamido]-3-/[(2,5-dihydro-6-hydroxy-2-methyl-5-oxo-as-triazin-3-yl)-thio]-methyl/-8-oxo-5-thia-1-azabicyclo-[4.2.0]oct-2-en-2-carbonsäure-Dinatriumsalz

Produkt B:
(6R, 7R)-7-[2-(2-Amino-4-thiazolyl)-2-(Z-hydroxyimino)acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure-Natriumsalz

Produkt C:
Methylen-(6R, 7R)-7-[2-(2-Amino-4-thiazolyl)-2-(Z-hydroxyimino)acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]-oct-2-en-2-carboxylat-pivalat)

Die nachstehenden Ergebnisse beziehen sich auf die Mindesthemmkonzentration (MHK) in vitro (μg/ml):

| Produkt A, Test in vitro | | MHK (μg/ml) |
|---|---|---|
| Staphylococcus aureus | | |
| | Stamm 1 | 0,62 |
| | Stamm 2: β-Lactamase erzeugend | 1,2 |
| Escherichia coli | Stamm 1 | 1,2 |
| | Stamm 2 | 1,2 |
| | Stamm 3 | 2,5 |
| | Stamm 4 | 0,31 |
| Klebsiella pneumoniae | Stamm 1 | 0,02 |
| Serratia marcescens | | |
| | Stamm 1 | 0,16 |
| | Stamm 2 | 0,31 |
| | Stamm 3 | 0,31 |
| | Stamm 4 | 0,63 |
| | Stamm 5 | 0,31 |
| | Stamm 6 | 0,31 |

| Produkt A, Test in vitro | | MHK (µg/ml) |
|---|---|---|
| Serratia marcescens | | |
| | Stamm 7 | 0,31 |
| | Stamm 8 | 0,31 |
| | Stamm 9 | 0,16 |
| Enterobacter cloacae | | |
| | Stamm 1 | 20 |
| | Stamm 2 | >80 |
| Citrobacter freundii | Stamm 1 | 80 |
| Proteus vulgaris | | |
| | Stamm 1 | 0,31 |
| | Stamm 2 | 0,31 |
| | Stamm 3 | 0,63 |
| Proteus morganii | Stamm 1 | ≤0,04 |
| Proteus rettgeri | Stamm 1 | 0,63 |
| Proteus inconstans | Stamm 1 | 0,08 |
| Proteus mirabilis | Stamm 1 | 0,02 |
| Pseudomonas aeruginosa | | |
| | Stamm 1 | >80 |
| | Stamm 2 | >80 |
| | Stamm 3 | 40 |
| | Stamm 4 | 80 |
| | Stamm 5 | >80 |
| | Stamm 6 | 10 |
| | Stamm 7 | 5 |
| | Stamm 8 | 20 |
| | Stamm 9 | >80 |
| | Stamm 10 | 10 |
| | Stamm 11 | 5 |
| | Stamm 12 | >80 |
| | Stamm 13 | 80 |
| | Stamm 14 | >80 |
| | Stamm 15 | 80 |
| | Stamm 16 | >80 |
| | Stamm 17 | >80 |
| Neisseria meningitidis | | |
| | Stamm 1 | 0,0025 |
| | Stamm 2 | 0,0025 |
| | Stamm 3 | 0,0025 |
| | Stamm 4 | 0,0012 |
| Neisseria gonorrhoeae | | |
| | Stamm 1 | 0,0012 |
| | Stamm 2 | 0,0012 |
| | Stamm 3 | 0,02 |
| | Stamm 4 | 0,01 |
| | Stamm 5 | 0,04 |
| | Stamm 6 | 0,0006 |
| Haemophilus influenzae | | |
| | Stamm 1 | 0,63 |
| | Stamm 2 | 0,04 |
| | Stamm 3 | 0,04 |
| | Stamm 4 | 0,04 |
| | Stamm 5 | 0,04 |
| | Stamm 6 | 0,02 |
| | Stamm 7 | 0,04 |

| Produkte A und B / Test in vitro | | MHK, µg/ml A | B |
|---|---|---|---|
| Staphylococcus aureus | Stamm 3 | 1,6 | 0,4 |
| | Stamm 4 | 1,6 | 0,8 |
| | Stamm 5 | 1,6 | 0,4 |
| | Stamm 6 | 3,1 | 0,8 |
| Staphylococcus epidermidis | | 1,6 | 0,4 |
| Streptococcus faecalis | Stamm 1 | 25 | 6,3 |
| | Stamm 2 | >25 | >25 |
| Streptococcus pyogenes | Stamm 1 | 0,1 | 0,025 |
| | Stamm 2 | 0,05 | 0,025 |
| Streptococcus pneumoniae | | 0,4 | 0,1 |
| Streptococcus viridans | | 1,6 | 0,4 |
| Escherichia coli | Stamm 5 | 0,4 | 0,4 |
| | Stamm 6 | 0,2 | 0,2 |
| | Stamm 7 | 0,025 | 0,05 |
| | Stamm 8 | 0,05 | 0,8 |
| Proteus mirabilis | Stamm 2 | 0,025 | 0,4 |
| | Stamm 3 | 0,05 | 1,6 |
| | Stamm 4 | 0,025 | 0,8 |
| Proteus inconstans | Stamm 2 | 0,1 | 0,2 |
| | Stamm 3 | ≤0,025 | ≤0,025 |
| Proteus vulgaris | Stamm 4 | 3,1 | 12,5 |
| | Stamm 5 | 0,1 | 0,8 |
| | Stamm 6 | 0,05 | 0,8 |
| Proteus morganii | Stamm 2 | 0,05 | 1,6 |
| Proteus rettgeri | Stamm 2 | 0,05 | 0,4 |
| Klebsiella pneumoniae | Stamm 2 | 0,1 | 1,6 |
| | Stamm 3 | 0,025 | 0,4 |
| | Stamm 4 | 0,05 | 0,8 |
| Serratia marcescens | Stamm 10 | 0,8 | 6,3 |
| | Stamm 11 | 6,3 | 12,5 |
| | Stamm 12 | 0,1 | 1,6 |
| Enterobacter cloacae | Stamm 3 | >25 | >25 |
| | Stamm 4 | 12,5 | 6,3 |
| Enterobacter aerogenes | Stamm 1 | 0,1 | 0,2 |
| | Stamm 2 | 0,8 | 1,6 |
| Citrobacter freundii | Stamm 2 | 6,3 | 3,1 |
| | Stamm 3 | 0,2 | 0,4 |

Zum Nachweis der langen Halbwertzeit der erfindungsgemässen Produkte wurden Ratten 20 mg des Produkts A pro kg i.v. verabreicht. Es wurde eine Halbwertzeit von 140 Minuten festgestellt.

Der lange Verbleib der erfindungsgemässen Produkte im Organismus wird auch ersichtlich nach dem s.c. Behandeln von Mäusen mit den Produkten A bzw. C 5 Stunden vor einer i.p.-Infektion des zu prüfenden Mikroorganismus, wobei die nachstehenden $ED_{50}$-Werte ermittelt werden:

| Erreger | $ED_{50}$ mg/kg s.c. A | C |
|---|---|---|
| Streptococcus pyogenes | 0,15 | 0,45 |
| Serratia marcescens | 0,89 | |
| Escherichia coli | | 5,6* |

* Verabreicht 24 Stunden vor der Infektion

Die erfindungsgemässen Verbindungen sind untoxisch, wie die nachstehenden Daten für die 24-Stunden-Werte der Produkte A und B zeigen:

| Applikationsart | Tödliche Dosis mg/kg A | Tolerierte Dosis mg/kg A | B |
|---|---|---|---|
| Oral | >5000 | 5000 | >5000 |
| Subcutan | >4000 | | >4000 |
| Intravenös | 1000 | 500 | |

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Öle, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung osmotischen Druckes, Anaesthetica oder Puffer. Sie können auch noch andere therapeutisch wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonische Kochsalzlösung, zubereitet. Die leicht hydrolysierbaren Ester bzw. Äther der Verbindungen der Formel I und ihre Salze bzw. Hydrate kommen auch für die enterale, z.B. orale Verabreichung in Betracht.

In DE-A-2 727 753 und FR-A-2 408 612 werden Cephalosporinverbindungen mit einer 2-(2-Amino-4-thiazolyl)-2-hydroxyimino-acetamido-Gruppe in 7-Stellung offenbart. Andererseits werden in EP-A-5830 Cephalosporinderivate mit einer
1,2,5,6-Tetrahydro-2-methyl-5,6-dioxo-as-triazin-3-yl- bzw.
2,5-Dihydroxy-6-hydroxy-2-methyl-5-oxo-as-triazin-3-yl-Gruppe
in 3-Stellung offenbart. Die Kombination dieser beiden Gruppen in 7- bzw. 3-Stellung wird erfindungsgemäss durch die Cephalosporine der vorliegenden Formel I bzw. ihre leicht hydrolysierbare Ester/Äther und Salze zustandegebracht.

Beispiel 1

Herstellung eines Dinatriumsalzes der
(6R, 7R)-7-[2-(2-Amino-4-thiazolyl)-2-(Z-hydroxyimino)acetamido]-3-/[(2,5-dihydro-6-hydroxy-2-methyl-5-oxo-as-triazin-3-yl)-thio]methyl/-8-oxo-5-thia-1-azabicyclo-[4.2.0]oct-2-en-2-carbonsäure:

10 g (6R, 7R)-7-[4-Brom-2-(Z-hydroxyimino)-acetoacetamido]-8-oxo-3-([(1,2,5,6-tetrahydro-2-methyl-5,6-dioxo-as-triazin-3-yl)-thio]methyl/-5-thia-1-azabicyclo[4.2.0]-oct-2-en-2-carbonsäure
werden in 50 ml Aceton gelöst und zu einer Lösung von 3,5 g Thioharnstoff in 150 ml Aceton gegeben. Das dabei sofort ausfallende Hydrobromid wird abgenutscht und mit Aceton und tiefsiedendem Petroläther gewaschen. Man erhält rosa Hydrobromid als Rohprodukt. Zwecks Überführung in das Dinatriumsalz wird es in einem Gemisch von 100 ml Wasser und 100 ml Methanol gelöst und mit 40 ml einer 2N Lösung von 2-Äthylcapronsäure-Natriumsalz in Essigester versetzt. Die orangefarbene Lösung wird nach Zusatz von Aktivkohle 1 Stunde bei 25 °C gerührt, dann filtriert und das Filtrat mit Methanol bis auf ein Volumen von 2 l verdünnt. Von wenig ausgefallenem Material (Fraktion 1, braun) wird abfiltriert. Das Filtrat wird am Vakuum bei 40 °C auf ca. 800 ml eingeengt und ausgefallene Substanz (Fraktion 2, gelb) abfiltriert. Das Filtrat wird auf ca. 200 ml eingeengt, mit 1 l Methanol verdünnt, wobei Substanz ausfällt, welche abfiltriert wird (Fraktion 3, gelb). Das Filtrat wird am Vakuum bei 40 °C auf ein Volumen von ca. 300 ml eingeengt und ausgefallene Substanz abfiltriert (Fraktion 4, gelb). Das Filtrat wird mit 1 l Äthanol versetzt und das amorph ausgefallene Material abfiltriert (Fraktion 5, beige). Nach dünnschichtchromatographischer und Kernresonanz-Analyse sind die Fraktionen 2, 3 und 4 identisch und stellen die gewünschte Verbindung dar; die Fraktionen 1 und 5 sind hingegen starke Gemische und werden verworfen. Nach dem Trocknen der Fraktionen 2, 3 und 4 im Hochvakuum bei 40 °C über Nacht erhält man gelbe, amorphe Titelverbindung mit $[\alpha]_D^{25} = -133{,}2°$ (c = 1 in Wasser). Die Substanz enthält 4% Wasser und 3% Methanol.

Die im obigen Verfahren als Ausgangsmaterial verwendete
(6R, 7R)-7-[4-Brom-2-(Z-hydroxyimino)-acetoacetamido]-8-oxo-3-/[(1,2,5,6-tetrahydro-2-methyl-5,6-dioxo-as-triazin-3-yl)thio]-methyl/-5-thia-1-azabicyclo[4.2.0]oct-2-en-2-carbonsäure
kann wie folgt hergestellt werden:

5,44 g Diketen werden in 25 ml Dichlormethan gelöst und bei −30 bis −40 °C mit einer Lösung von 10,4 g Brom in 25 ml Dichlormethan tropfenweise während etwa 15 Minuten versetzt. Die farblose Lösung wird auf −50 °C gekühlt und während etwa 10 Minuten bei −20 °C einer Lösung zugetropft, die durch Versetzen von 18,5 g
(7R)-7-Amino-3-desacetoxy-3-[(2,5-dihydro-6-hydroxy-2-methyl-5-oxo-as-triazin-3-yl)-thio]-cephalosporansäure
in 300 ml abs. Tetrahydrofuran mit 50 ml N,O-Bis-(trimethylsilyl)-acetamid während 30 Minuten hergestellt wurde. Das Reaktionsgemisch wird 30 Minuten ohne Kühlung gerührt, wobei die Temperatur auf 0 °C steigt. Das orangebraune Gemisch wird dann auf 2 l Äthylacetat gegossen und mit 500 ml Wasser kräftig durchgerührt. Die dabei gebildete Zwischenschicht wird abfiltriert und verworfen. Die orangefarbene organische Phase wird dreimal mit je 500 ml Wasser gewaschen und anschliessend, ohne getrocknet zu werden, mit 10 g Aktivkohle 1½ Stunden gerührt. Nach dem Abfiltrieren der Kohle wird das hellgelbe Filtrat über Natriumsulfat getrocknet und am Vakuum bei 40 °C auf ein Volumen von ca. 100 ml eingeengt. Die dabei auskristallisierte Substanz wird abgenutscht und mit wenig Äthylacetat gewaschen. Man erhält reine, beigefarbene
(6R, 7R)-7-(4-Bromacetoacetamido)-8-oxo-3-/[(1,2,5,6-tetrahydro-2-methyl-5,6-dioxo-as-triazin-3-yl)thio]methyl/-5-thia-1-azabicyclo[4.2.0]oct-2-en-2-carbonsäure.
Aus der Mutterlauge wird durch Fällen mit Äther weitere farblose, amorphe Reinsubstanz gewonnen. $[\alpha]_D^{25} = -247{,}3°$ (c = 1 in Dimethylformamid).

46,6 g (6R, 7R)-7-(4-Bromacetoacetamido)-8-oxo-3-/[(1,2,5,6-tetrahydro-2-methyl-5,6-dioxo-as-triazin-3-yl)thio]methyl/-5-thia-1-azabicyclo[4.2.0]oct-2-en-2-carbonsäure
werden in 100 ml Eisessig suspendiert und bei 10–15 °C mit einer Lösung von 2,5 g Natriumnitrit in 10 ml Wasser während etwa 15 Minuten versetzt. Das Reaktionsgemisch wird 1½ Stunden bei 10 °C gerührt. Die so entstandene, orangefarbene Lösung wird auf 1 l Äthylacetat gegossen, einmal mit 600 ml 0,16N Schwefelsäure, dann dreimal mit je 500 ml Wasser gewaschen, über Natriumsulfat getrocknet und am Vakuum bei 40 °C stark eingeengt. Das Konzentrat wird mit Äther versetzt, wobei die Substanz amorph ausfällt. Diese wird abgenutscht, nacheinander mit Äther und tiefsiedendem Petroläther gewaschen und im Vakuum bei 25 °C getrocknet. Man erhält beigefarbene
(6R, 7R)-7-[4-Brom-2-(Z-hydroxyimino)-acetoacetamidol-8-oxo-3-/[(1,2,5,6-tetrahydro-2-methyl-5,6-dioxo-as-triazin-3-yl)thio]-methyl/-5-thia-1-azabicyclo[4.2.0]oct-2-en-2-carbonsäure
mit $[\alpha]_D^{20} = -279{,}2°$ (c = 1 in Dimethylformamid).

Beispiel 2

Herstellung des Natriumsalzes der
(6R, 7R)-7-[2-(2-Amino-4-thiazolyl)-2-(Z-hydroxyimino)acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure:

5,4 g (6R, 7R)-7-[4-Brom-2-(Z-hydroxyimino)-acetoacetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure
werden in 70 ml Äthanol suspendiert und mit 1,2 g Thioharnstoff versetzt. Die entstandene gelbe Lösung wird 25 Minuten bei 25 °C gerührt. Dann werden 9 ml einer 2N Lösung von 2-Äthylcapronsäure-Natriumsalz in Äthylacetat hinzugefügt, wobei die Substanz in sehr feiner Form ausfällt. Nach Zugabe von 300 ml Methanol und 130 ml Äthanol wird die leicht trübe Lösung filtriert und das hellgelbe Filtrat im Vakuum bei 40° auf ein Volumen von ca. 100 ml eingeengt. Das dabei ausgefallene Material wird abgenutscht, mit Äthanol und tiefsiedendem Petroläther gewaschen und über Nacht im Hochvakuum bei 40 °C getrocknet. Man erhält reine, beigefarbene, amorphe Titelverbindung mit $[\alpha]_D^{25} = -133{,}3°$ (c = 1 in Wasser). Das Kernresonanzspektrum und die Mikroanalyse entsprechen der angegebenen Struktur.

Die in obigem Verfahren als Ausgangsmaterial verwendete
(6R, 7R)-7-[4-Brom-2-(Z-hydroxyimino)aceto-acetamido]-3-[[[2,5-dihydro-2-methyl-5-oxo-6-[(pivaloyloxy)methoxy]-as-triazin-3-yl]-thio]methyl]-8-oxo-5-thia-1-azabicyclo-[4.2.0]oct-2-en-2-carbonsäure
kann wird wie folgt hergestellt werden:

a) Herstellung von Tetrahydro-2-methyl-5,6-dioxo-3-thioxo-as-triazin-1(2H)-carbonsäure-benzylester:

39,79 g 1,2,5,6-Tetrahydro-5,6-dioxo-3-mercapto-2-methyl-as-triazin
werden in 500 ml Dimethylformamid und mit 35 ml Triäthylamin versetzt. Während 15 Minuten werden 39 ml Benzyloxycarbonylchlorid zugetropft, wobei die Temperatur des Reaktionsgemisches auf 45 °C steigt, Triäthylamin-hydrochlorid ausfällt und die Suspension gelb gefärbt wird. Das Gemisch wird 2½ Stunden bei 25 °C gerührt und anschliessend am Vakuum bei 70 °C eingedampft. Der ölige Eindampfrückstand wird mit 500 ml Wasser während 1 Stunde verrührt, wobei dieser fest wird. Der erhaltene Festkörper wird abgenutscht und mit 50 ml Wasser gewaschen. Das gelbe Nutschgut wird mit 250 ml Äthanol gut verrührt, abgenutscht, mit Äthanol gewaschen und im Vakuum bei 40 °C getrocknet. Man erhält farbloses Rohkristallisat, das aus Methanol umkristallisiert wird und farblose Reinsubstanz vom Smp. 150–153 °C liefert.

b) Herstellung von 3-[(Diphenylmethyl)-thio]-5,6-dihydro-2-methyl-5,6-dioxo-as-triazin-1(2H)-carbonsäure-benzylester:

13,2 g Tetrahydro-2-methyl-5,6-dioxo-3-thioxo-as-triazin-1(2H)-carbonsäure-benzylester werden in 500 ml Essigester gelöst und mit einer Lösung von Diphenyldiazomethan in 90 ml tiefsiedendem Petroläther versetzt. Die anfangs violette Reaktionslösung wird 40 Stunden bei 25 °C stehen gelassen, wobei die Farbe blassrot wird. Es werden 3 ml Eisessig zugegeben, und das Gemisch wird nach 1 Stunde im Vakuum bei 40 °C eingedampft. Man erhält ein gelbes Öl als Eindampfrückstand. Dieser wird mittels Säulenchromatographie an Kieselgel mit den Elutionsmitteln Benzol, Benzol/Äthylacetat 95 : 5 und Benzol/Äthylacetat 90 : 10 aufgetrennt. Die die gewünschte Substanz enthaltenden Fraktionen werden gesammelt und im Vakuum bei 40 °C eingedampft. Man erhält, nach dem Umkristallisieren aus Äthanol, farblose Reinsubstanz vom Smp. 90–92 °C.

c) Herstellung von 3-[(Diphenylmethyl)-thio]-1,2-dihydro-2-methyl-as-triazin-5,6-dion:

6,9 g der unter b) hergestellten Substanz werden in 100 ml Äthylacetat gelöst und mit 30 ml einer wässrigen, 7%igen Ammoniak-Lösung bei 25 °C während 15 Minuten gut durchgerührt. Die Äthylacetatphase wird abgetrennt und verworfen. Die wässrige Phase wird mit 7 ml konz. Salzsäure versetzt und 30 Minuten im Eisbad gerührt. Die dabei ausgefallene Substanz wird abgenutscht, mit Wasser gewaschen und sofort aus Äthanol umkristallisiert. Man erhält farblose Reinsubstanz vom Smp. 180–182 °C.

d) Herstellung von 3-[(Diphenylmethyl)-thio]-6-pivaloyloxy-methoxy-2-methyl-as-triazin-6(2H)-on:

3,25 g der unter c) hergestellten Verbindung werden in 40 ml Dimethylformamid gelöst und mit 1,52 g Kaliumcarbonat versetzt. Zu diesem Gemisch werden auf einmal 2,66 g Pivaloyloxymethyljodid gegeben, wobei eine blassgelbe Lösung entsteht. Das Reaktionsgemisch wird 4 Stunden bei 25 °C gerührt und noch einmal mit 2,66 g Pivaloyloxymethyljodid versetzt. Dieses Gemisch wird 15 Stunden bei 25 °C gerührt und anschliessend im Hochvakuum bei 35 °C eingedampft. Der resultierende Eindampfrückstand wird zwischen 100 ml Wasser und 100 ml Äthylacetat verteilt. Die wässrige Phase wird noch zweimal mit je 50 ml Äthylacetat extrahiert. Die vereinigten Äthylacetat-Phasen werden über Natriumsulfat getrocknet und im Vakuum bei 35 °C eingedampft. Das verbleibende gelbe Öl wird an einer Kieselgelsäule mit Äthylacetat als Elutionsmittel chromatographiert. Die das gewünschte Produkt enthaltenden Fraktionen werden gesammelt und im Vakuum bei 35 °C eingedampft. Das verbleibende blassblaue Öl wird im Hochvakuum bei 25 °C während 1 Stunde getrocknet, wobei man die Reinsubstanz als blassblaues Harz enthält.

e) Herstellung von [(2,3,4,5-Tetrahydro-2-methyl-5-oxo-3-thioxo-as-triazin-6-yl)-oxy]methyl pivalat:

3,6 g der unter d) hergestellten Verbindung werden in 18 ml Anisol und 18 ml Trifluoressigsäure 2½ Stunden bei 25 °C gerührt. Die Lösung wird dann im Vakuum bei 50 °C eingedampft. Der ölige Rückstand wird mit 50 ml tiefsiedendem Petroläther verrührt, wobei Kristallisation eintritt. Das Kristallisat wird abgenutscht und mit tiefsiedendem Petroläther gewaschen. Man erhält weisse Kristalle, die nach dem Umkristallisieren aus Äther/tiefsiedendem Petroläther weisse, kristalline Reinsubstanz vom Smp. 112–113 °C liefern. Das Kernresonanzspektrum und die Mikroanalyse entsprechen der angegebenen Struktur.

f) Herstellung von (6R, 7R)-7-Amino-3-[[[2,5-dihydro-2-methyl-5-oxo-6-[pivaloyloxy)methoxy]-as-triazin-3-yl]-thio]-methyl]-8-oxo-5-thia-1-azabicyclo[4.2.0]-oct-2-en-2-carbonsäure:

3,0 g der unter e) hergestellten Verbindung werden zusammen mit 2,72 g 7-Aminocephalosporansäure in 50 ml Wasser suspendiert. Die Suspension wird unter Stickstoff-Begasung mit 1N Natronlauge auf pH 6,5 gestellt und 4 Stunden bei pH 6,5–7 und 55–60 °C gerührt. Die gewünschte Verbindung fällt aus und wird nach dem Abkühlen des Gemisches auf 25 °C abgenutscht. Nach dem Waschen mit Wasser, Aceton und tiefsiedendem Petroläther und Trocknen über Nacht im Hochvakuum bei 40 °C erhält man die Reinsubstanz, deren Mikroanalyse und Kernresonanzspektrum der angegebenen Struktur entsprechen.

g) Herstellung von (6R, 7R)-7-[4-Brom-acetoacetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure:

Zunächst werden 4,35 g Diketen in 20 ml Methylenchlorid gelöst und bei −30 bis −40 °C mit einer Lösung von 8,32 g Brom in 20 ml Methylenchlorid tropfenweise während ca. 15 Minuten versetzt. Die das Säurebromid enthaltende, farblose Lösung wird auf −50 °C gekühlt und während ca. 10 Minuten zu einer folgendermassen hergestellten Lösung bei −20 °C zugetropft: 19,42 g der unter f) hergestellten Verbindung werden in 400 ml Äthylacetat suspendiert und mit 40 ml N,O-Bis-(trimethylsilyl)-acetamid versetzt. Die Suspension wird 30 Minuten bei 35–40 °C gerührt, bis eine bräunliche Lösung entstanden ist. Diese wird zur Acylierung mit dem wie oben hergestellten Säurebromid auf −20 °C gekühlt. Nach dem Zutropfen der Säurebromid-Lösung wird das Reaktionsgemisch noch 1 Stunde ohne Kühlung gerührt. Danach werden 200 ml Wasser zugegeben. Das ausgefallene, nicht umgesetzte Ausgangsmaterial wird abgenutscht und verworfen. Die wässrige Phase wird abgetrennt. Die orangefarbene organische Phase wird zweimal mit je 250 ml Wasser gewaschen, über Natriumsulfat getrocknet und im Vakuum bei 40 °C eingeengt. Nach Zugabe von Äther fällt das Reaktionsprodukt amorph aus. Es wird abgenutscht, mit Äther und tiefsiedendem Petroläther gewaschen und im Vakuum bei 25 °C getrocknet. Man erhält rohe amorphe Substanz, die zwecks Reinigung aus Äthylacetat/Äther umgefällt wird. Man erhält somit die beigefarbene Titelverbindung mit $[\alpha]_D^{25} = -159,3°$ (c = 1 in Chloroform).

h) Herstellung von (6R, 7R)-7-[4-Brom-2-(Z-hydroxyimino)-acetoacetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-en-2-carbonsäure:

13 g der unter g) hergestellten Verbindung werden in 65 ml Essigsäure gelöst. Diese Lösung wird bei 10–15 °C während ca. 45 Minuten mit einer Lösung von 1,65 g Natriumnitrit in 7 ml Wasser tropfenweise versetzt. Die Reaktionslösung wird 1¼ Stunden bei 10–15 °C nachgerührt, bis keine

salpetrige Säure mittels Jodkaliumstärkepapier mehr nachweisbar ist. Die rötliche Lösung wird dann auf 500 ml Äthylacetat gegossen. Dieses Gemisch wird zweimal mit verdünnter Natriumchlorid-Lösung gewaschen. Die organische Phase wird mit Natriumsulfat getrocknet und im Vakuum bei 40 °C stark eingeengt. Der verbleibende sirupöse Rückstand wird mit Äther versetzt, wobei die Substanz amorph ausfällt. Diese wird abgenutscht, mit Äther und mit tiefsiedendem Petroläther gewaschen und über Nacht im Vakuum bei 40 °C getrocknet. Man erhält ein bräunliches, amorphes Pulver, welches zwecks Reinigung in 100 ml Isopropanol verrührt wird, wobei zunächst eine Lösung entsteht, aus welcher sich dann eine gallertartige Masse abscheidet, die nach ca. 45 Minuten Rühren bei 25 °C kristallisiert. Die kristalline Substanz wird abgenutscht, mit wenig Isopropanol, Äther und tiefsiedendem Petroläther gewaschen und im Hochvakuum bei 40 °C getrocknet. Man erhält die weisse, kristalline Reinsubstanz mit $[\alpha]_D^{25} = -284{,}6°$ (c = 1 in Chloroform). Das Kernresonanzspektrum entspricht der angegebenen Struktur.

Beispiel 3

Herstellung von Methylen-(6R, 7R)-7-[2-(-Amino-4-thiazolyl)-2-(Z-hydroxyimino)acetamido]-3-[[[2,5-dihydro-2-methyl-5-oxo-6[(pivaloyloxy)methoxy]-as-triazin-3-yl]-thio]-methyl]-8-oxo-5-thia-1-azabicyclo[4.2.0]-oct-2-en-2-carboxylat-pivalat:

1,35 g Natriumsalz der
(6R, 7R)-7-[2-(2-Amino-4-thiazolyl)-2-(Z-hydroxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure
werden in 25 ml Dimethylformamid gelöst und mit 1 g Pivaloyloxymethyljodid bei 0–5 °C versetzt. Das Gemisch wird 30 Minuten bei 0–5 °C unter Stickstoff-Begasung gerührt. Danach wird es auf 400 ml Äthylacetat gegossen, nacheinander zweimal mit je 100 ml Wasser, zweimal mit je 50 ml 8%iger Natriumhydrogencarbonat-Lösung, einmal mit 100 ml 5%iger Natriumthiosulfat-Lösung und zweimal mit je 100 ml Wasser gewaschen und schliesslich über Natriumsulfat getrocknet. Die Äthylacetatlösung wird dann im Vakuum bei 40 °C eingeengt und mit 150 ml Äther versetzt, wobei die Substanz amorph ausfällt. Diese wird abgenutscht, mit Äther und tiefsiedendem Petroläther gewaschen und über Nacht im Hochvakuum bei 40–45 °C getrocknet. Man erhält beigefarbene Reinsubstanz mit $R_f = 0{,}57$ im DC-System Butanol/Essigsäure/Wasser 4 : 1 : 1 auf Kieselgel-$F_{254}$-Fertigplatten. Das Kernresonanzspektrum entspricht der angegebenen Struktur.

Beispiel 4

Herstellung von Trockenampullen für die intramuskuläre Verabreichung:

Es wird in üblicher Weise ein Lyophilisat von 1 g des Dinatriumsalzes der
(6R, 7R)-7-[2-(2-Amino-4-thiazolyl)-2-(Z-hydroxyimino)acetamido]-3-/[(2,5-dihydro-6-hydroxy-2-methyl-5-oxo-as-triazin-3-yl)-thio]methyl/-8-oxo-5-thia-1-azabicyclo-[4.2.0]oct-2-en-2-carbonsäure
hergestellt und in eine Ampulle abgefüllt. Vor der Verabreichung wird das Lyophilisat mit 2,5 ml einer 2%igen wässrigen Lidocainhydrochlorid-Lösung versetzt.

Beispiel 5

Es wird in üblicher Weise eine Gelatine-Steckkapsel folgender Zusammensetzung hergestellt:

| | |
|---|---|
| Methylen-(6R, 7R)-7-[2-(2-Amino-4-thiazolyl)-2-(Z-hydroxyimino)acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carboxylat-pivalat | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

**Patentansprüche für die Vertragsstaaten BE CH DE FR GB IT LI LU NL SE**

1. Acylderivate der allgemeinen Formel

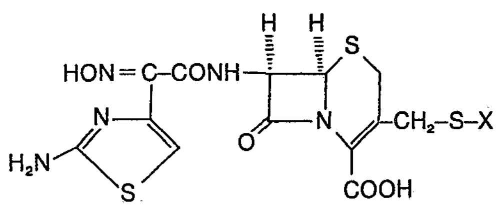

I

worin X die 1,2,5,6-Tetrahydro-2-methyl-5,6-dioxo-as-triazin-3-ylgruppe bzw. deren entsprechende tautomere Form, die 2,5-Dihydro-6-hydroxy-2-methyl-5-oxo-as-triazin-3-ylgruppe, darstellt, sowie leicht hydrolysierbare Ester, leicht hydrolysierbare Äther und Salze dieser Verbindungen und Hydrate der Verbindungen der Formel I bzw. von deren Estern, Äthern und Salzen.

2. Acylderivate nach Anspruch 1 in der synisomeren Form bzw. Gemische, in denen die syn-isomere Form überwiegt.

3. Die Carbonsäuren der Formel 1 nach Anspruch 1 oder 2, sowie Salze dieser Verbindungen und Hydrate dieser Verbindungen bzw. Salze.

4. (6R, 7R)-7-[2-(2-Amino-4-thiazolyl)-2-(Z-hydroxyimino)-acetamido]-3-/[2,5-dihydro-6-hydroxy-2-methyl-5-oxo-as-triazin-3-yl)-thio]-methyl/-8-oxo-5-thia-1-azabicyclo-[4.2.0]oct-2-en-2-carbonsäure
sowie Salze dieser Verbindung und Hydrate dieser Verbindung bzw. Salze.

5. Leicht hydrolysierbare Äther und Acylderivate der Formel I gemäss einem der Ansprüche 1–4 sowie Salze dieser Äther und Hydrate dieser Äther und Salze.

6. Pivaloyloxymethyläther der Acylderivate der Formel I gemäss Anspruch 5, sowie Salze dieser Äther und Hydrate dieser Äther und Salze.

7. (6R, 7R)-7-[2-(2-Amino-4-thiazolyl)-2-(Z-hydroxyimino)acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-en-2-carbonsäure sowie Salze dieser Äther und Hydrate dieser Äther und Salze.

8. Leicht hydrolysierbare Ester der Acylderivate der Formel I gemäss einem der Ansprüche 1–4 sowie Salze dieser Ester und Hydrate dieser Ester und Salze.

9. Pivaloyloxymethylester der Acylderivate der Formel I gemäss Anspruch 8 sowie Salze dieser Ester und Hydrate dieser Ester und Salze.

10. Methylen(6R, 7R)-7-[2-(2-amino-4-thiazolyl)-2-(Z-hydroxyimino)-acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carboxylat-pivalat sowie Salze dieser Verbindung und Hydrate dieser Verbindung und Salze.

11. Acylderivate der allgemeinen Formel

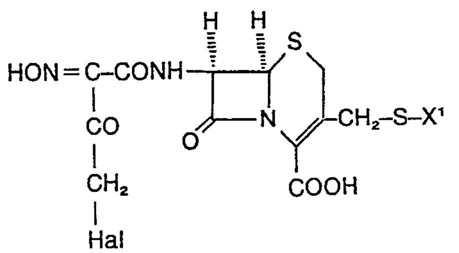

II

in der $X^1$ die 1,2,5,6-Tetrahydro-2-methyl-5,6-dioxo-as-triazin-3-ylgruppe oder eine gegebenenfalls zu einem leicht hydrolysierbaren Äther verätherte 2,5-Dihydro-6-hydroxy-2-methyl-5-oxo-as-triazin-3-ylgruppe, Hal Brom oder Chlor darstellt und die Carboxygruppe in geschützter Form vorliegen kann.

12. Acylderivate gemäss Anspruch 11, dadurch gekennzeichnet, dass $X^1$ die 2,5-Dihydro-6-hydroxy-2-methyl-5-oxo-as-triazin-3-ylgruppe darstellt.

13. Acylderivate gemäss Anspruch 11 oder 12, dadurch gekennzeichnet, dass Hal Brom darstellt.

14. (6R, 7R)-7-[4-Brom-2-(Z-hydroxyimino)-acetoacetamido]-8-oxo-3-/[(1,2,5,6-tetrahydro-2-methyl-5,6-dioxo-as-triazin-3-yl)-thio]methyl/-5-thia-1-azabicyclo[4.2.0]oct-2-en-2-carbonsäure.

15. (6R, 7R)-7-[4-Brom-2-(Z-hydroxyimino)-acetoacetamido]-3-[[[2,5-dihydro-2-methyl-5-oxo-6[(pivaloyloxy)methoxy]-as-triazin-3-yl]thio]methyl]-8-oxo-5-thia-1-azabicyclo-[4.2.0]oct-2-en-2-carbonsäure.

16. Verbindungen gemäss einem der Ansprüche 1–10 als pharmazeutische Wirkstoffe.

17. Verbindungen gemäss einem der Ansprüche 1–10 als pharmazeutische Wirkstoffe zur Behandlung und Prophylaxe von Infektionskrankheiten.

18. Pharmazeutische Präparate, gekennzeichnet durch einen Gehalt an einer Verbindung gemäss einem der Ansprüche 1–10.

19. Verfahren zur Herstellung der Verbindungen gemäss einem der Ansprüche 1–10, dadurch gekennzeichnet, dass man

a) eine Verbindung der allgemeinen Formel

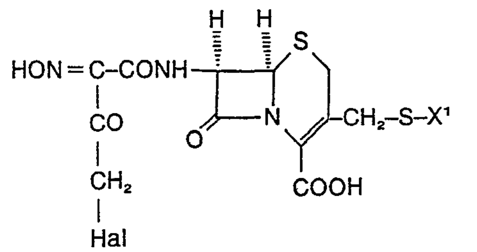

II

in der $X^1$ dieselbe Bedeutung wie X in Anspruch 1 hat, wobei jedoch die 2,5-Dihydro-6-hydroxy-2-methyl-5-oxo-as-triazin-3-ylgruppe zu einem leicht hydrolysierbaren Äther veräthert sein kann, Hal Brom oder Chlor darstellt und die Carboxygruppe in geschützer Form vorliegen kann, mit Thioharnstoff umsetzt und eine allenfalls vorhandene Carboxyschutzgruppe abspaltet, oder dass man

b) zur Herstellung eines leicht hydrolysierbaren Esters bzw. Äthers einer Verbindung der Formel I eine Carbonsäure bzw. ein Enol der Formel I einer entsprechenden Veresterung bzw. Verätherung unterwirft, oder dass man

c) zur Herstellung von Salzen oder Hydraten einer Verbindung der Formel I bzw. Hydraten dieser Salze eine Verbindung der Formel I in ein Salz oder ein Hydrat bzw. ein Hydrat dieses Salzes überführt.

**Patentansprüche für den Vertragsstaat: AT**

1. Verfahren zur Herstellung von Acylderivaten der allgemeinen Formel

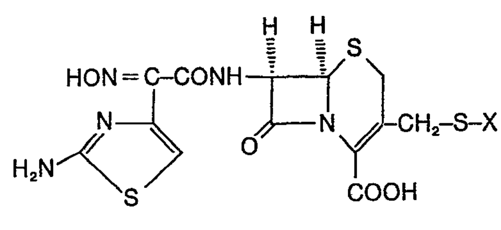

I

worin X die
1,2,5,6-Tetrahydro-2-methyl-5,6-dioxo-as-triazin-3-ylgruppe
bzw. deren entsprechende tautomere Form, die
2,5-Dihydro-6-hydroxy-2-methyl-5-oxo-as-triazin-3-ylgruppe,
darstellt,
sowie von leicht hydrolysierbaren Estern, leicht hydrolysierbaren Äthern und von Salzen dieser Verbindungen sowie vom Hydraten der Verbindungen der Formel I bzw. von deren Estern, Äthern und Salzen, dadurch gekennzeichnet, dass man

a) eine Verbindung der allgemeinen Formel

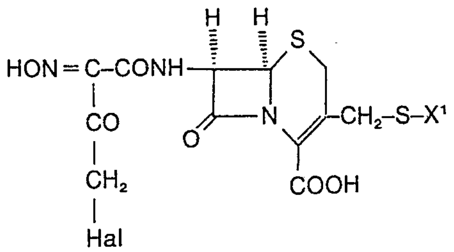

II

in der $X^1$ dieselbe Bedeutung wie X in Formel I hat, wobei jedoch die 2,5-Dihydro-6-hydroxy-2-methyl-5-oxo-as-triazin-3-ylgruppe zu einem leicht hydrolysierbaren Äther veräthert sein kann, Hal Brom oder Chlor darstellt und die Carboxygruppe in geschützter Form vorliegen kann, mit Thioharnstoff umsetzt und eine allenfalls vorhandene Carboxyschutzgruppe abspaltet, oder dass man

b) zur Herstellung eines leicht hydrolysierbaren Esters bzw. Äthers einer Verbindung der Formel I eine Carbonsäure bzw. ein Enol der Formel I einer entsprechenden Veresterung bzw. Verätherung unterwirft, oder dass man

c) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

2. Verfahren nach Anspruch 1 zur Herstellung von Acylderivaten nach Anspruch 1 in der synisomeren Form bzw. Gemische, in denen die synisomere Form überwiegt, dadurch gekennzeichnet, dass man Ausgangsverbindungen mit entsprechender Konfiguration einsetzt.

3. Verfahren nach Anspruch 1 oder 2 zur Herstellung von Carbonsäuren der Formel I nach Anspruch 1 oder 2, sowie von Salzen dieser Verbindungen und von Hydraten dieser Verbindungen bzw. Salze, dadurch gekennzeichnet, dass man entsprechend substituierte Ausgangsverbindungen einsetzt.

4. Verfahren nach Anspruch 3 zur Herstellung von

(6R, 7R)-7-[2-(2-Amino-4-thiazolyl)-2-(Z-hydroxyimino)-acetamido]-3-/[2,5-dihydro-6-hydroxy-2-methyl-5-oxo-as-triazin-3-yl)-thio]-methyl/-8-oxo-5-thia-1-azabicyclo-[4.2.0]oct-2-en-2-carbonsäure

sowie von Salzen dieser Verbindung und von Hydraten dieser Verbindung bzw. Salze, dadurch gekennzeichnet, dass man entsprechend substituierte Ausgangsverbindungen einsetzt.

5. Verfahren nach Anspruch 1 oder 2 zur Herstellung von leicht hydrolysierbaren Äthern der Acylderivate der Formel I gemäss einem der Ansprüche 1–4 sowie von Salzen dieser Äther und von Hydraten dieser Äther und Salze, dadurch gekennzeichnet, dass man entsprechend substituierte Ausgangsverbindungen einsetzt.

6. Verfahren nach Anspruch 5 zur Herstellung von Pivaloyloxymethyläthern der Acylderivate der Formel I gemäss Anspruch 5 sowie von Salzen dieser Äther und von Hydraten dieser Äther und Salze, dadurch gekennzeichnet, dass man entsprechend substituierte Ausgangsverbindungen einsetzt.

7. Verfahren nach Anspruch 6 zur Herstellung von

(6R, 7R)-7-[2-(2-Amino-4-thiazolyl)-2-(Z-hydroxyimino)-acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-en-2-carbonsäure

sowie von Salzen dieser Äther und von Hydraten dieser Äther und Salze, dadurch gekennzeichnet, dass man entsprechend substituierte Ausgangsverbindungen einsetzt.

8. Verfahren nach Anspruch 1 oder 2 zur Herstellung von leicht hydrolysierbaren Estern der Acylderivate der Formel I gemäss einem der Ansprüche 1–4 sowie von Salzen dieser Ester und von Hydraten dieser Ester und Salze, dadurch gekennzeichnet, dass man entsprechend substituierte Ausgangsverbindungen einsetzt.

9. Verfahren nach Anspruch 8 zur Herstellung von Pivaloyloxymethylestern der Acylderivate der Formel I gemäss Anspruch 8 sowie von Salzen dieser Ester und von Hydraten dieser Ester und Salze, dadurch gekennzeichnet, dass man entsprechend substituierte Ausgangsverbindungen einsetzt.

10. Verfahren nach Anspruch 9 zur Herstellung von

Methylen(6R, 7R)-7-[2-(2-amino-4-thiazolyl)-2-(Z-hydroxyimino)-acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-en-2-carboxylat-pivalat

sowie von Salzen dieser Verbindung und von Hydraten dieser Verbindung und Salze, dadurch gekennzeichnet, dass man entsprechend substituierte Ausgangsverbindungen einsetzt.

**Claims for the Contracting States: BE CH DE FR GB IT LI LU NL SE**

1. Acyl derivatives of the general formula

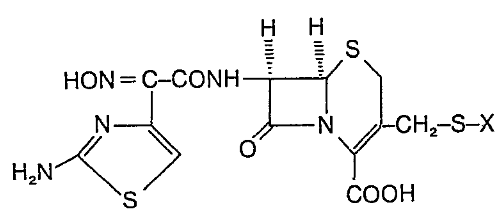

I

wherein X represents the
1,2,5,6-tetrahydro-2-methyl-5,6-dioxo-as-triazin-3-yl group
or its corresponding tautomeric form, the
2,5-dihydro-6-hydroxy-2-methyl-5-oxo-as-triazin-3-yl group,
as well as readily hydrolyzable esters, readily hydrolyzable ethers and salts of these compounds and hydrates of the compounds of formula I or of their esters, ethers and salts.

2. Acyl derivatives according to claim 1 in the syn-isomeric form or mixtures in which the syn-isomeric form predominates.

3. The carboxylic acids of formula I according to claim 1 or 2, as well as salts of these compounds and hydrates of these compounds or salts.

4. (6R, 7R)-7-[2-(2-Amino-4-thiazolyl)-2-(Z-hydroxyimino)-acetamido]-3-/[2,5-dihydro-6-hydroxy-2-methyl-5-oxo-as-triazin-3-yl)-thio]-methyl/-8-oxo-5-thia-1-azabicyclo-[4.2.0]oct-2-ene-2-carboxylic acid
as well as salts of this compound and hydrates of this compound or salts.

5. Readily hydrolyzable ethers of the acyl derivatives of formula I in accordance with any one of claims 1–4 as well as salts of these ethers and hydrates of these ethers and salts.

6. Pivaloyloxymethyl ethers of the acyl derivatives of formula I in accordance with claim 5 as well as salts of these ethers and hydrates of these ethers and salts.

7. (6R, 7R)-7-[2-(2-Amino-4-thiazolyl)-2-(Z-hydroxyimino)acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-ene-2-carboxylic acid
as well as salts of this ether and hydrates of this ether and salts.

8. Readily hydrolyzable esters of the acyl derivatives of formula I in accordance with any one of claims 1–4 as well as salts of these esters and hydrates of these esters and salts.

9. Pivaloyloxymethyl esters of the acyl derivatives of formula I in accordance with claim 8 as well as salts of these esters and hydrates of these esters and salts.

10. Methylene (6R, 7R)-7-[2-(2-amino-4-thiazolyl)-2-(Z-hydroxyimino)acetamido]-3-[[[2,5-dihydro-2-methyl-5-oxo-6-[(pivaloyl-oxy)-methoxy]-as-triazin-3-yl]thio]methyl]-8-oxo-5-thia-1-azabicyclo[4.2.0]oct-2-ene-2-carboxylate pivalate
as well as salts of this compound and hydrates of this compound and salts.

11. Acyl derivatives of the general formula

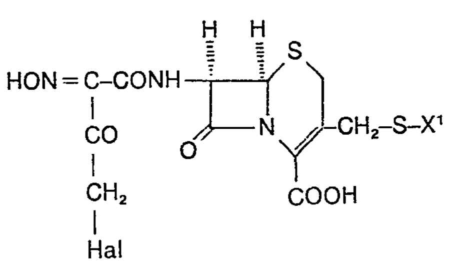

II

in which $X^1$ represents the
1,2,5,6-tetrahydro-2-methyl-5,6-dioxo-as-triazin-3-yl group or a 2,5-dihydro-6-hydroxy-2-methyl-5-oxo-as-triazin-3-yl group
optionally etherified to a readily hydrolyzable ether, Hal represents bromine or chlorine and the carboxy group can be present in protected form.

12. Acyl derivatives in accordance with claim 11, characterized in that $X^1$ represents the 2,5-dihydro-6-hydroxy-2-methyl-5-oxo-as-triazin-3-yl group.

13. Acyl derivatives in accordance with claim 11 or 12, characterized in that Hal represents bromine.

14. (6R, 7R)-7-[4-Bromo-2-(Z-hydroxyimino)-acetoacetamido]-8-oxo-3-/[(1,2,5,6-tetrahydro-2-methyl-5,6-dioxo-as-triazin-3-yl)-thio]methyl/-5-thia-1-azabicyclo[4.2.0]oct-2-ene-2-carboxylic acid.

15. (6R, 7R)-7-[4-Bromo-2-(Z-hydroxyimino)-acetoacetamido]-3-[[[2,5-dihydro-2-methyl-5-oxo-6[(pivaloyloxy)-methoxy]-as-triazin-3-yl]thio]methyl]-8-oxo-5-thia-1-aza-bicyclo[4.2.0]oct-2-ene-2-carboxylic acid.

16. Compounds in accordance with any one of claims 1–10 as pharmaceutically active substances.

17. Compounds in accordance with any one of claims 1–10 as pharmaceutically active substances for the treatment and prophylaxis of infectious diseases.

18. Pharmaceutical preparation, characterized by a content of a compound in accordance with any one of claims 1–10.

19. A process for the manufacture of the compounds in accordance with any one of claims 1–10, characterized by

a) reacting a compound of the general formula

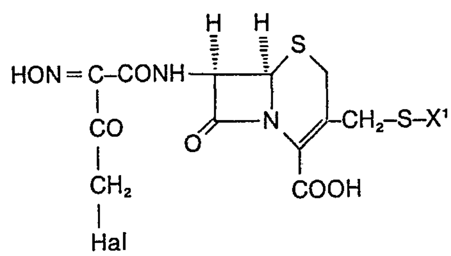

II

in which $X^1$ has the same significance as X in claim 1, whereby, however, the
2,5-dihydro-6-hydroxy-2-methyl-5-oxo-as-triazin-3-yl group
can be etherified to a readily hydrolyzable ether, Hal represents bromine or chlorine and the carboxy group can be present in protected form,
with thiourea and cleaving off a carboxy protecting group which may be present, or by

b) for the manufacture of a readily hydrolyzable ester or ether of a compound of formula I, subjecting a carboxylic acid or an enol of formula I to a corresponding esterification or etherification, or by

c) for the manufacture of salts or hydrates of a compound of formula I or hydrates of these salts, converting a compound of formula I into a salt or hydrate or into a hydrate of this salt.

**Claims for the Contracting State: AT**

1. A process for the manufacture of acyl derivatives of the general formula

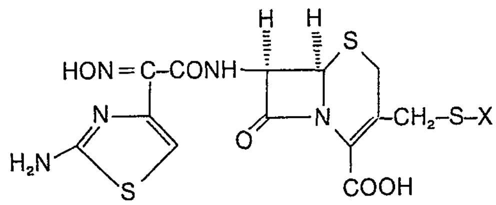

I

wherein X represents the
1,2,5,6-tetrahydro-2-methyl-5,6-dioxo-as-triazin-3-yl group
or its corresponding tautomeric form, the
2,5-dihydro-6-hydroxy-2-methyl-5-oxo-as-triazin-3-yl group,
as well as of readily hydrolyzable esters, readily hydrolyzable ethers and of salts of these compounds as well as of hydrates of the compounds of formula I or of their esters, ethers and salts, characterized by

a) reacting a compound of the general formula

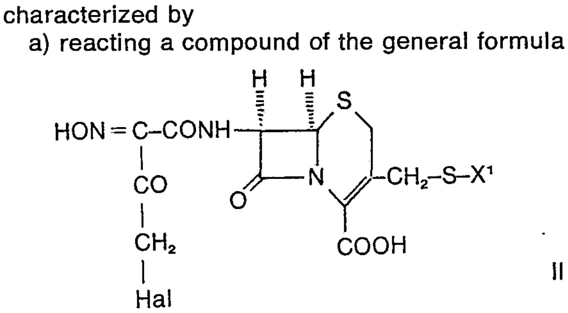

II

in which X¹ has the same significance as X in formula I, whereby, however, the 2,5-dihydro-6-hydroxy-2-methyl-5-oxo-as-triazin-3-yl group can be etherified to a readily hydrolyzable ether, Hal represents bromine or chlorine and the carboxy group can be present in protected form, with thiourea and cleaving off a carboxy protecting group which may be present, or by

b) for the manufacture of a readily hydrolyzable ester or ether of a compound of formula I, subjecting a carboxylic acid or an enol of formula I to a corresponding esterification or etherification, or by

c) for the manufacture of salts or hydrates of a compound of formula I or hydrates of these salts, converting a compound of formula I into a salt or hydrate or into a hydrate of this salt.

2. A process according to claim 1 for the manufacture of acyl derivatives according to claim 1 in the syn-isomeric form or mixtures in which the syn-isomeric form predominates, characterized by using starting compounds with the corresponding configuration.

3. A process according to claim 1 or 2 for the manufacture of carboxylic acids of formula I according to claim 1 or 2, as well as of salts of these compounds and of hydrates of these compounds or salts, characterized by using correspondingly substituted starting compounds.

4. A process according to claim 3 for the manufacture of
(6R, 7R)-7-[2-(2-amino-4-thiazolyl)-2-(Z-hydroxyimino)-acetamido]-3-/[2,5-dihydro-6-hydroxy-2-methyl-5-oxo-as-triazin-3-yl)-thio]-methyl/-8-oxo-5-thia-1-azabicyclo-[4.2.0]oct-2-ene-2-carboxylic acid
as well as of salts of this compound and of hydrates of this compound or salts, characterized by using correspondingly substituted starting compounds.

5. A process according to claim 1 or 2 for the manufacture of readily hydrolyzable ethers of the acyl derivatives of formula I in accordance with any one of claims 1–4 as well as of salts of these ethers and of hydrates of these ethers and salts, characterized by using correspondingly substituted starting compounds.

6. A process according to claim 5 for the manufacture of pivaloyloxymethyl ethers of the acyl derivatives of formula I in accordance with claim 5 as well as of salts of these ethers and of hydrates of these ethers and salts, characterized by using correspondingly substituted starting compounds.

7. A process according to claim 6 for the manufacture of
(6R, 7R)-7-[2-(2-amino-4-thiazolyl)-2-(Z-hydroxyimino)acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-ene-2-carboxylic acid
as well as of salts of this ether and of hydrates of this ether and salts, characterized by using correspondingly substituted starting compounds.

8. A process according to claim 1 or 2 for the manufacture of readily hydrolyzable esters of the acyl derivatives of formula I in accordance with any one of claims 1–4 as well as of salts of these esters and of hydrates of these esters and salts, characterized by using correspondingly substituted starting compounds.

9. A process according to claim 8 for the manufacture of pivaloyloxymethyl esters of the acyl derivatives of formula I in accordance with claim 8 as well as of salts of these esters and of hydrates of these esters and salts, characterized by using correspondingly substituted starting compounds.

10. A process according to claim 9 for the manufacture of methylene
(6R, 7R)-7-[2-(2-amino-4-thiazolyl)-2-(Z-hydroxyimino)-acetamido]-3-[[[2,5-dihydro-2-methyl-5-oxo-6-[(pivaloyloxy)-methoxy]-as-triazin-3-yl]thio]methyl]-8-oxo-5-thia-1-azabicyclo[4.2.0]oct-2-ene-2-carboxylate pivalate
as well as of salts of this compound and of hydrates of this compound and salts, characterized by using correspondingly substituted starting compounds.

**Revendications pour les Etats contractants: BE, CH, DE, FR, GB, IT, LI, LU, NL, SE**

1. Dérivés acylés de formule générale

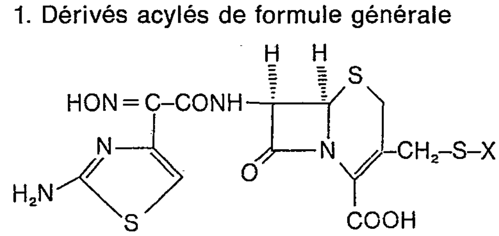

I

dans laquelle X représente le groupe
1,2,5,6-tétrahydro-2-méthyl-5,6-dioxo-as-triazine-3-yle
ou la forme tautomère correspondante, le groupe
2,5-dihydro-6-hydroxy-2-méthyl-5-oxo-as-triazine-3-yle,
et les esters faciles à hydrolyser, éthers faciles à hydrolyser et sels de ces composés et hydrates des composés de formule I et de leurs esters, éthers et sels.

2. Dérivés acylés selon la revendication 1, sous la forme isomère syn et mélanges dans lesquels la forme isomère syn est prépondérante.

3. Les acides carboxyliques de formule I selon la revendication 1 ou 2 et les sels de ces composés et hydrates de ces composés et sels.

4. L'acide (6R, 7R)-7-[2-(2-amino-4-thiazolyl)-2-(Z-hydroxyimino)-acétamido]-3-/[2,5-dihydro-6-hydroxy-2-méthyl-5-oxo-as-triazine-3-yl)-thio]-méthyl/-8-oxo-5-thia-1-azabicyclo[4.2.0]octa-2-ène-2-carboxylique
et les sels de ce composé et hydrates de ce composé et de ses sels.

5. Ethers faciles à hydrolyser des dérivés acylés de formule I selon l'une des revendications 1 à 4 et sels de ces éthers et hydrates de ces éthers et sels.

6. Ethers pivaloyloxyméthyliques des dérivés acylés de formule I selon la revendication 5 et sels de ces éthers et hydrates de ces éthers et sels.

7. L'acide (6R, 7R)-7-[2-(2-amino-4-thiazolyl)-2-(Z-hydroxyimino)-acétamido]-3-[[[2,5-dihydro-2-méthyl-5-oxo-6-[(pivaloyloxy)-méthoxy]-as-triazine-3-yl]-thio]-méthyl]-8-oxo-5-thia-1-azabicyclo[4.2.0]-octa-2-ène-2-carboxylique
et les sels de ces éthers et hydrates de ces éthers et sels.

8. Esters faciles à hydrolyser des dérivés acylés de formule I selon l'une des revendications 1 à 4, et les sels de ces esters et hydrates de ces esters et sels.

9. Esters pivaloyloxyméthyliques des dérivés acylés de formule I selon la revendication 8 et les sels de ces esters et hydrates de ces esters et sels.

10. Le (6R, 7R)-7-[2-(2-amino-4-thiazolyl)-2-(Z-hydroxyimino)-acétamido]-3-[[[2,5-dihydro-2-méthyl-5-oxo-6-[(pivaloyloxy)-méthoxy]-as-triazine-3-yl]-thio]-méthyl]-8-oxo-5-thia-1-azabicyclo[4.2.0]octa-2-ène-2-carboxylate-pivalate de méthylène
et les sels de ce composé et hydrates de ce composé et de ses sels.

11. Dérivés acylés de formule générale

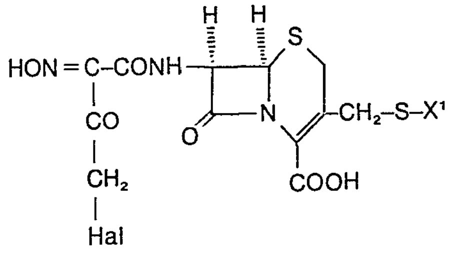

II

dans laquelle $X^1$ représente le groupe
1,2,5,6-tétrahydro-2-méthyl-5,6-dioxo-as-triazine-3-yle
ou un groupe
2,5-dihydro-6-hydroxy-2-méthyl-5-oxo-as-triazine-3-yle
éventuellement éthérifié sous forme d'un éther facile à hydrolyser, Hal représente le brome ou le chlore et le groupe carboxy peut être à l'état protégé.

12. Dérivés acylés selon la revendication 11, caractérisés en ce que $X^1$ représente le groupe 2,5-dihydro-6-hydroxy-2-méthyl-5-oxo-as-triazine-3-yle.

13. Dérivés acylés selon la revendication 11 ou 12, caractérisés en ce que Hal représente le brome.

14. L'acide (6R, 7R)-7-[4-bromo-2-(Z-hydroxyimino)-acétoacétamido]-8-oxo-3-/[(1,2,5,6-tétrahydro-2-méthyl-5,6-dioxo-as-triazine-3-yl)-thio]-méthyl/-5-thia-1-azabicyclo[4.2.0]octa-2-ène-2-carboxylique.

15. L'acide (6R,7R)-7-[4-bromo-2-(Z-hydroxyimino)-acétoacétamido]-3-[[[2,5-dihydro-2-méthyl-5-oxo-6-[(pivaloyloxy)-méthoxy]-as-triazine-3-yl]-thio]-méthyl]-8-oxo-5-thia-1-azabicyclo[4.2.0]octa-2-ène-2-carboxylique.

16. Les composés selon l'une des revendications 1 à 10, en tant que substances actives pharmaceutiques.

17. Les composés selon l'une des revendications 1 à 10, en tant que substances actives pharmaceutiques pour le traitement et la prophylaxie de maladies infectieuses.

18. Compositions pharmaceutiques caractérisées en ce qu'elles contiennent un composé selon l'une des revendications 1 à 10.

19. Procédé de préparation des composés selon l'une des revendications 1 à 10, caractérisé en ce que:

a) on fait réagir un composé de formule générale

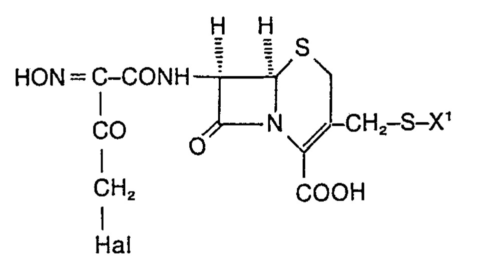

II

dans laquelle $X^1$ a les mêmes significations que X de la revendication 1, le groupe
2,5-dihydro-6-hydroxy-2-méthyl-5-oxo-as-triazine-3-yle
pouvant toutefois être éthérifié sous la forme d'un éther facile à hydrolyser, Hal représente le brome ou le chlore et le groupe carboxy peut être à l'état protégé, avec la thiourée et on élimine un groupe protecteur éventuel du groupe carboxy, ou bien

b) pour la préparation d'un ester ou éther facile à hydrolyser d'un composé de formule I, on soumet un acide carboxylique ou un énol respectivement de formule I à estérification ou éthérification respectivement, ou bien

c) pour préparer des sels ou hydrates d'un composé de formule I ou des hydrates de ces sels, on convertit un composé de formule I en un sel ou hydrate ou respectivement ce sel et un hydrate.

**Revendications pour l'Etat contractant: AT**

1. Procédé de préparation de dérivés acylés de formule générale

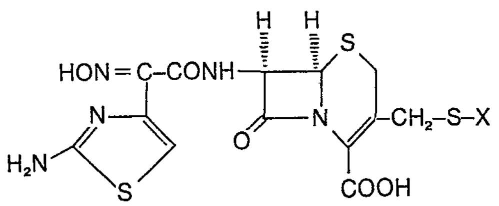

I

dans laquelle X représente le groupe
1,2,5,6-tétrahydro-2-méthyl-5,6-dioxo-as-triazine-3-yle
ou la forme tautomère correspondante, le groupe
2,5-dihydro-6-hydroxy-2-méthyl-5-oxo-as-triazine-3-yle,
et de leurs esters faciles à hydrolyser, de leurs éthers faciles à hydrolyser et des sels de ces composés ainsi que des hydrates des composés de formule I et de leurs ester, éthers et sels, caractérisé en ce que:

a) on fait réagir un composé de formule générale

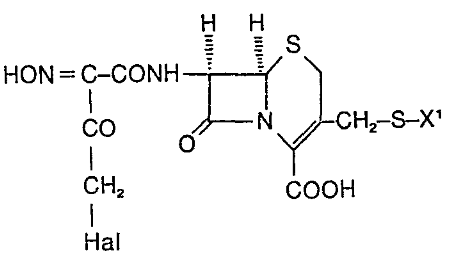

II

dans laquelle $X^1$ a les mêmes significations que X de la formule I, le groupe
2,5-dihydro-6-hydroxy-2-méthyl-5-oxo-as-triazine-3-yle
pouvant toutefois être éthérifié sous la forme d'un éther facile à hydrolyser, Hal représente le brome ou le chlore et le groupe carboxy peut être à l'état protégé,
avec la thiourée et on élimine un groupe protecteur éventuel du groupe carboxy, ou bien

b) pour la préparation d'un ester ou éther facile à hydrolyser d'un composé de formule I, on soumet respectivement un acide carboxylique ou un énol de formule I à estérification ou éthérification respectivement, ou bien

c) pour préparer les sels ou hydrates d'un composé de formule I ou les hydrates de ces sels, on convertit un composé de formule I en un sel ou hydrate ou ce sel en un hydrate.

2. Procédé selon la revendication 1, pour la préparation des dérivés acylés selon la revendication 1 sous la forme isomère syn ou de mélanges dans lesquels la forme isomère syn est prédominante, caractérisé en ce que l'on met en œuvre des composés de départ ayant la configuration correspondante.

3. Procédé selon la revendication 1 ou 2, pour préparer les acides carboxyliques de formule I selon la revendication 1 ou 2, ainsi que les sels de ces composés et les hydrates de ces composés et sels, caractérisé en ce que l'on met en œuvre des composés de départ portant les substituants correspondants.

4. Procédé selon la revendication 3, pour préparer l'acide
(6R,7R)-7-[2-(2-amino-4-thiazolyl)-2-(Z-hydroxyimino)-acétamido]-3-/[2,5-dihydro-6-hydroxy-2-méthyl-5-oxo-as-triazine-3-yl)-thio]-méthyl]-8-oxo-5-thia-1-azabicyclo[4.2.0]octa-2-ène-2-carboxylique
et les sels de ce composé et les hydrates de ce composé et de ses sels, caractérisé en ce que l'on met en œuvre des composés de départ portant les substituants correspondants.

5. Procédé selon la revendication 1 ou 2, pour préparer des éthers faciles à hydrolyser des dérivés acylés de formule I selon l'une des revendications 1 à 4, ainsi que les sels de ces éthers et les hydrates de ces éthers et sels, caractérisé en ce que l'on met en œuvre des composés de départ portant les substituants correspondants.

6. Procédé selon la revendication 5, pour préparer les éthers pivaloyloxyméthyliques des dérivés acylés de formule I selon la revendication 5, ainsi que les sels de ces éthers et les hydrates de ces éthers et sels, caractérisé en ce que l'on met en œuvre des composés de départ portant les substituants correspondants.

7. Procédé selon la revendication 6, pour préparer l'acide
(6R,7R)-7-[2-(2-amino-4-thiazolyl)-2-(Z-hydroxyimino)-acétamido]-3-[[[2,5-dihydro-2-méthyl-5-oxo-6-[(pivaloyloxy)-méthoxy]-as-triazine-3-yl]-thio]-méthyl]-8-oxo-5-thia-1-azabicyclo[4.2.0]-octa-2-ène-2-carboxylique
et les sels de ces éthers et hydrates de ces éthers et sels, caractérisé en ce que l'on met en œuvre des composés de départ portant les substituants correspondants.

8. Procédé selon la revendication 1 ou 2, pour préparer des esters faciles à hydrolyser des dérivés acylés de formule I selon l'une des revendications 1 à 4, ainsi que les sels de ces esters et les hydrates de ces esters et sels, caractérisé en ce que l'on met en œuvre des composés de départ portant les substituants correspondants.

9. Procédé selon la revendication 8, pour préparer les esters pivaloyloxyméthyliques des dérivés acylés de formule I selon la revendication 8, ainsi que les sels de ces esters et les hydrates de ces esters et sels, caractérisé en ce que l'on met en œuvre des composés de départ portant les substituants correspondants.

10. Procédé selon la revendication 9, pour préparer le (6R,7R)-7-[2-(2-amino-4-thiazolyl)-2-(Z-hydroxyimino)-acétamido]-3-[[[2,5-dihydro-2-méthyl-5-oxo-6-[(pivaloyloxy)-méthoxy]-as-triazine-3-yl)-thio]-méthyl]-8-oxo-5-thia-1-azabicyclo[4.2.0]octa-2-ène-2-carboxylate-pivalate de méthylène et les sels de ce composé et les hydrates de ce composé et de ses sels, caractérisé en ce que l'on met en œuvre des composés de départ portant les substituants correspondants.